## Europäisches Patentamt
## European Patent Office
## Office européen des brevets

(19)

(11) Publication number: **0 044 543**
**B1**

(12) **EUROPEAN PATENT SPECIFICATION**

(45) Date of publication of patent specification: **06.06.84**　(51) Int. Cl.³: **A 61 K 9/10, A 61 K 31/52**

(21) Application number: **81105635.7**

(22) Date of filing: **17.07.81**

(54) Topical formulations of 9-(2-hydroxyethoxymethyl) guanine.

| | |
|---|---|
| (30) Priority: **18.07.80 GB 8023645**<br>**15.10.80 GB 8033218** | (73) Proprietor: **THE WELLCOME FOUNDATION LIMITED**<br>**183-193 Euston Road**<br>**London NW1 2BP (GB)** |
| (43) Date of publication of application:<br>**27.01.82 Bulletin 82/4** | |
| | (72) Inventor: **Jones, Trevor Mervyn**<br>**48 Glebe Hyrst**<br>**Sanderstead Surrey (GB)**<br>Inventor: **White, Alan Robert**<br>**136 Timberbank Vigo Village**<br>**Meopham Kent (GB)** |
| (45) Publication of the grant of the patent:<br>**06.06.84 Bulletin 84/23** | |
| (84) Designated Contracting States:<br>**BE CH DE FR GB LI LU NL SE** | |
| | (74) Representative: **Berg, Wilhelm, Dr. et al,**<br>**Dr. Berg, Dipl.-Ing. Stapf, Dipl.-Ing. Schwabe, Dr.**<br>**Dr. Sandmair Mauerkircherstrasse 45**<br>**D-8000 München 80 (DE)** |
| (56) References cited:<br>**DE - A - 2 606 516**<br>**DE - A - 2 847 975**<br>**FR - A - 2 207 692**<br>**FR - A - 2 213 023**<br>**FR - A - 2 222 998**<br>**FR - A - 2 316 948**<br>**GB - A - 1 495 692**<br>**US - A - 3 934 013**<br>**US - A - 4 141 976**<br>**US - A - 4 199 574** | |

Courier Press, Leamington Spa, England.

## Description

This invention relates to a topical pharmaceutical formulation suitable for use in treating virus infections of the skin and mucosa, and in particular it relates to topical formulations containing 9 - (2 - hydroxyethoxymethyl)guanine, otherwise known as acyclovir, and hereinafter referred to as such.

Acyclovir and pharmaceutically acceptable salts and esters thereof are known to have antiviral activity against various classes of DNA and RNA viruses both *in vitro* and *in vivo*, see UK patent No 1 523 865. In particular the compound is active against herpes simplex virus which causes herpetic keratitis in rabbits, herpetic encephalitis in mice, and cutaneous herpes in guinea pigs.

Acyclovir suffers from the disadvantage that it has a low solubility in water and is almost totally insoluble in hydrophobic solvent systems. It is accordingly difficult to produce a topical formulation containing a sufficient dissolved concentration of active ingredient for it to exert its full effect and also to optimise the flux of the compound into the skin. In addition to ease of release it is also important that any formulation of a pharmaceutically active compound should be stable for long periods of time, should not lose its potency, should not discolour or form insoluble substances or complexes, and also should not be unduly irritating to the skin or mucosa.

In example 26 of UK patent No 1 523 865 there are listed the constituents of an oil-in-water cream containing 5% w/w acyclovir, amongst which constituents is 5% w/w propylene glycol. The function of the propylene glycol in the formulation of example 26 is to act as an humectant, i.e. a hygroscopic ingredient, which should improve the cosmetic feel of the product and also limit dehydration during storage. In animal experiments this formulation and a formulation of aqueous cream B.P. (British Pharmacoepia) containing acyclovir did not provide a particularly rapid cure probably because of insufficient active ingredient in solution and poor penetration of the active ingredient into the skin.

In view of the lipid nature of the skin surface, especially the stratum corneum, it has long been thought that to achieve good transdermal penetration the active ingredient in an emulsion should be located in the oil phase so that it can partition into the lipid components of the skin.

In French Patent Specification 2,207,692, there are described various topical formulations containing an anti-inflammatory steroid together with an aqueous phase containing propylene glycol. However, the latter ingredient is a water-insoluble, fat-soluble material and thus differs considerably in its pharmaceutical behaviour from acyclovir which has a low (but definite) water-solubility and is fat-insoluble.

Other topical formulations of similar composition are described in French Patent Specification 2,316,946 and U.S. Patent Specification 3,934,013.

It has now been found that, in order to optimise the release of acyclovir from topical formulations, the maximum solubilised concentration of drug should be in the external phase of an oil-in-water emulsion preparation, i.e. in the aqueous phase. Further it has been found that by using a high concentration of a polyhydric alcohol as a cosolvent in the aqueous phase, for example at least 50% v/v of that phase, an increased concentration of solubilised acyclovir can be attained, leading to enhanced activity and efficacy of such formulations. Such a high concentration of a polyhydric alcohol also dispenses with the necessity of including a preservative as an additional ingredient in the formulation.

Such topical formulations also satisfy the criteria of adequate stability, maintenance of potency and are not unduly irritating to the skin or mucosa and have the advantages over the prior art formulation of penetrating skin more effectively and in greater concentration with the result that a rapid, complete cure of the infection is achieved.

According to the present invention there is provided an oil-in-water topical pharmaceutical formulation for the treatment of virus diseases of the skin or mucosa of a mammal comprising a dispersed oil phase and a continuous aqueous phase containing therein water, at least 30% of a water miscible polyhydric alcohol (by weight of the formulation) and solubilised acyclovir. Preferably the formulation contains a maximum of 50% water.

Such a topical formulation may contain 0.075% to 10% w/w acyclovir or a salt or an ester thereof, from 30% to 60% w/w of a polyhydric alcohol, from 15% to 50% w/w water and an oil phase. Hereafter references to acyclovir should be understood to include also its pharmaceutically acceptable salts and esters unless the context clearly indicates otherwise.

In a preferred aspect the formulation comprises from 1% to 10% w/w acyclovir, from 30% to 50% w/w of a polyhydric alcohol, from 20% to 40% w/w water together with an oil phase, whilst the most preferred formulation comprises from 2% to 5% w/w acyclovir, from 35% to 45% w/w of a polyhydric alcohol, from 25% to 40% w/w water together with an oil phase. The formulation should preferably contain about 40% w/w of a polyhydric alcohol.

A polyhydric alcohol is an alcohol having two or more hydroxyl groups. Polyhydric alcohols suitable for incorporation into the topical formulation of the present invention include glycols and macrogols such as propylene glycol, butane 1,3-diol, polyethylene glycol and glycerol, propylene glycol being the preferred alcohol.

When at least 50% v/v of a polyhydric alcohol is used in the aqueous phase of a formulation of the present invention, the maximum concentration of acyclovir at ambient temperature rises from 0.15% w/w, that being the maximum aqueous solubility of acyclovir, to 0.3% w/w. Thus if aqueous phase concentrations of greater than 0.3% w/w acyclovir are incorporated into a formulation the amount of active ingredient in excess of 0.3% will be in suspension and act as a reservoir of drug. The amount of a acyclovir present in the formulation should be at least sufficient to be antivirally effective and to be non-toxic. The water used in the formulation is preferably purified water, purified that is according to the standards of the British Pharmacopoeia.

The oil phase of the emulsions of this invention may be constituted from known ingredients in a known manner. While the phase may comprise merely an emulsifier (otherwise known as an emulgent), it is desirably comprised of a mixture of at least one emulsifier with a fat or an oil or with both a fat and an oil. Preferably, as explained in more detail below, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so called emulsifying ointment base which forms the oil dispersed phase of the emulsions.

Oil-in-water topical formulations may be formulated in a number of ways, all of which depend primarily on the alignment of the emulgent or emulsifying agent and emulsion stabiliser at the oil/water interface, with the non-polar or lipophilic groups solube in the oil phase and the polar or hydrophilic or lipophilic groups in the aqueous or continuous phase. Thus the more polar hydrophilic emulgents result in oil-in-water emulsions. This principle has been systemised in the idea of a "hydrophilic-lipophilic balance" (H.L.B.) Griffen, W. C, *J. Soc. Cos. Met. Chem.*, 1954, *5*, 249 and the various emulgents have been allocated H.L.B. numbers from which their behaviour with constituents of the aqueous and oil phases (to which are applied theoretical required H.L.B. figures) may be predicted.

It is a well established theory of oil-in-water emulsion formulation that the combination of a lipophilic emulgent with a hydrophilic emulgent of the same chemical type may be used in varying proportions to give the required H.L.B. value. With the high concentration of polyhydric alcohol required to maximise acyclovir release from the formulation of the present invention an H.L.B. value of from 3.5 to 10.0, preferably 4.0 to 8.0, most preferably about 5.5, is desirable, compared with the accepted H.L.B. range for mineral oil-in-water emulsions of 8 to 18.

Emulgents and emulsion stabilisers suitable for use in the formulation of the present invention include polyoxyethylene sorbitan monostearate (polysorbate 60), sorbitan monostearate, sorbitan mono-oleate, cetostearyl alcohol, myristyl alcohol, glyceryl monostearate and sodium lauryl sulphate. One preferred combination of emulgents is cetostearyl alcohol and sodium lauryl sulphate in a ratio of from 3:1 to 30:1 preferably from 6:1 to 20:1, most preferably from 9:1 to 15:1.

In addition, the formulation may optionally contain other emulgents such as poloxamers in an amount of from 0.1 to 3% w/w, preferably 0.3 to 2% w/w, most preferably about 1% w/w of the formulation.

The choice of suitable oils or fats for the formulation is based on acheiving the desired cosmetic properties, since the solubility of acyclovir in most oils likely to be used in pharmaceutical emulsion formulations is very low. Thus the cream should preferably be a nongreasy, non-staining and washable product with suitable consistency to avoid leakage from tubes or other containers. Straight or branched chain, mono- or dialkyl esters such as diisopropyl adipate, isocetyl stearate, propylene glycol diester of coconut fatty acids, isopropyl myristate, decyl oleate, isopropyl palmitate, butyl stearate, 2-ethylhexyl palmitate or a mixed ester of 2-ethyl hexanoic acid with a blend of cetyl or stearyl alcohols known as Crodamol CAP may be used, the last three being the preferred esters. These may be used singly or in combination depending on the properties required. Alternatively, high melting point lipids such as white soft paraffin and/or liquid paraffin or other mineral oils can be used.

The present invention further provides a method for the preparation of a topical pharmaceutical formulation, as hereinbefore defined, which comprises mixing the combination of acyclovir, polyhydric alcohol and water with the oil phase.

The manner of formulating the emulsion will of course vary according to the amount and nature of the constituents, but nevertheless follows known techniques in emulsion technology (see The Pharmaceutical Codex, London, The Pharmaceutical Press, 1979). For example the acyclovir may be initially incorporated wholly in the aqueous portion where it may form a solution alone, or a mixed solution/suspension, and then emulsified with the ointment base. Alternatively where high concentrations of acyclovir are being used, a part of the aqueous portion may be formulated as an emulsion, and the balance of the water, polyhydric alcohol and acyclovir added to and dispersed into the emulsion. In another technique the acyclovir may be included in the emulsifying ointment prior to emulsification with the aqueous portion. In using these procedures, it is preferable to heat the aqueous portion and the ointment base to about 40 to 80°C, preferably 50 to 70°C, prior to emulsification which may be achieved by vigorous agitation using for example a standard laboratory mixer. Finer dispersions of the oil phase may be obtained by homogenising or milling in a colloidal mill.

A topical formulation of the present invention may be used in the treatment or prevention of viral infections caused for example by Herpes zoster, Herpes varicella and Herpes simplex types 1 and 2, which cause diseases such as shingles, chicken pox, cold sores and genital herpes. The formulation should desirably be applied to the affected area of skin from 2 to 6 times daily, preferably from 3 to 4 times.

The following are examples of the invention.

Example 1
2% w/w Aqueous Cream
An aqueous cream was prepared from the following ingredients:

| | | |
|---|---|---|
| 1. Acyclovir | 20.0g |
| 2. Cetostearyl alcohol, B.P. | 67.5g |
| 3. Sodium lauryl sulphate, B.P. | 7.5g |
| 4. White soft paraffin, B.P. | 125.0g |
| 5. Liquid paraffin, B.P. | 50.0g |
| 6. Propylene glycol, B.P. | 400.0g |
| 7. Purified water, B.P. to | 1000.0g |

A part of the acyclovir (2g) was dissolved in the water and propylene glycol at ambient temperature to produce an aqueous solution. The paraffins (4, 5) and emulsifiers (2, 3) were mixed together and heated to 60°C, and emulsified with the aqueous solution, also at 60°C, using a laboratory mixer at 8000 r.p.m.. The remaining acyclovir was added, the mixture dispersed, allowed to cool, and filled into lacquered aluminium tubes.

Example 2
5% w/w Aqueous Cream
In the manner described above, an aqeuous cream was prepared containing 5% w/w acyclovir.

Example 3
0.2% w/w Aqueous Cream

| | | |
|---|---|---|
| 1. Acyclovir | 2.0g |
| 2. Isopropyl myristate, B.P. | 100.0g |
| 3. 2-Ethylhexyl palmitate | 50.0g |
| 4. Light liquid paraffin, B.P. | 50.0g |
| 5. Cetostearyl alcohol, B.P. | 30.0g |
| 6. Glyceryl monostearate, B.P. | 16.0g |
| 7. Polysorbate 60, B.P.C. | 4.0g |
| 8. Propylene glycol, B.P. | 400.0g |
| 9. Purified water, B.P. to | 1000.0g |

The cream was prepared in the manner described in Example 1 except that all the acyclovir was initially dissolved in the propylene glycol/water ingredients (8, 9).

Example 4
2% w/w Aqueous Cream
An aqueous cream was prepared from the following ingredients by the method described in Example 1.

| | | |
|---|---|---|
| 1. Acyclovir | 20.0g |
| 2. Cetostearyl alcohol, B.P. | 67.5g |
| 3. Sodium lauryl Sulphate, B.P. | 7.5g |
| 4. White soft paraffin, B.P. | 125.0g |
| 5. Liquid paraffin, B.P. | 50.0g |
| 6. Butane 1,3-diol, B.P. | 400.0g |
| 7. Purified water, B.P. to | 1000.0g |

**Claims**

1. An oil-in-water topical formulation of 9-(2-hydroxyethoxymethyl)guanine (hereinafter referred to as acyclovir) or a pharmaceutically acceptable salt and ester thereof, having a dispersed oil phase and a continuous aqueous phase characterised in that the aqueous phase comprises water, at least 30% w/w, based on the total weight of the formulation, of a water miscible polyhydric alcohol and solubilized acyclovir or salt or ester thereof.

2. A formulation as claimed in claim 1 comprising from 1% to 10% w/w acyclovir or a salt or ester, from 30% to 50% w/w of the polyhydric alcohol, from 20% to 40% w/w water, all percentages based on the total weight of the formulation, together with the oil phase.

3. A formulation as claimed in claim 1 or claim 2 comprising from 2% to 5% w/w acyclovir or a salt or ester, from 35% to 45% w/w of the polyhydric alcohol, from 25% to 40% w/w water, all percentages based on the total weight of the formulation, together with the oil phase.

4. A formulation as claimed in any one of claims 1 to 3 comprising about 40% w/w, based on the total weight of the formulation, of the polyhydric alcohol.

5. A formulation as claimed in any one of the preceding claims wherein the polyhydric alcohol is a glycol or macrogol.

6. A formulation as claimed in claim 5 wherein glycol or macrogol is propylene glycol, polyethylene glycol, butane 1,3-diol or glycerol.

7. A formulation as claimed in any one of the preceding claims wherein the oil phase comprises at least one emulsifier.

8. A formulation as claimed in claim 7 wherein the emulsifier is cetostearyl alcohol, myristyl alcohol, glyceryl monostearate, sodium lauryl sulphate, polyoxyethylene sorbitan monostearate, sorbitan monostearate or sorbitan mono-oleate.

9. A formulation as claimed in claim 7 wherein the oil phase also comprises a fat and/or an oil.

10. A formulation as claimed in claim 9 wherein the fat and/or oil are di-isopropyl adipate, isocetyl stearate, propylene glycol diester of coconut fatty acids, isopropyl myristate, decyl oleate, isopropyl palmitate, butyl stearate, 2-ethylhexyl palmitate, white soft paraffin, liquid paraffin, mineral oils or a mixed ester of 2-ethylhexanoic acid with a blend of cetyl and stearyl alcohols.

11. An oil-in-water topical formulation of 9-(2-hydroxyethoxymethyl)guanine or a pharmaceutically acceptable salt and ester thereof, having a dispersed oil phase and a continuous aqueous phase characterized in that the aqueous phase comprises water, at least 30% w/w, based on the total weight of the formulation, of propylene glycol and solubilized acyclovir or salt or ester thereof.

12. A method of preparing a topical formulation as claimed in any one of claims 1 to 11 comprising mixing the combination of acyclovir, polyhydric alcohol and water with the oily phase.

**Patentansprüche**

1. Äußerlich anzuwendende Öl-in-Wasser-Formulierung von 9-(2-Hydroxyethoxymethyl)-guanin (nachstehend als Acyclovir bezeichnet) oder eines pharmazeutisch verträglichen Salzes oder Esters davon, die eine dispergierte Ölphase und eine kontinuierliche wässrige Phase aufweist, dadurch gekennzeichnet, daß die wässrige Phase Wasser, mindestens 30 Gew.-%, basierend auf dem Gesamtgewicht der Formulierung, eines mit Wasser mischbaren Alkohols mit mehreren Hydroxylgruppen und solubilisiertes Acyclovir oder dessen Salz oder dessen Ester enthält.

2. Formulierung nach Anspruch 1, enthaltend 1 bis 10 Gew.-% Acyclovir oder ein Salz oder einen Ester davon, 30 bis 50 Gew.-% des Alkohols mit mehreren Hydroxylgruppen, 20 bis 40 Gew.-% Wasser, wobei alle Prozentangaben auf dem Gesamtgewicht der Formulierung basieren, zusammen mit der Ölphase.

3. Formulierung nach Anspruch 1 oder Anspruch 2, enthaltend 2 bis 5 Gew.-% Acyclovir oder ein Salz oder einen Ester davon, 35 bis 45 Gew.-% des Alkohols mit mehreren Hydroxylgruppen, 25 bis 40 Gew.-% Wasser, wobei alle Prozentgehalte auf dem Gesamtgewicht der Formulierung basieren, zusammen mit der Ölphase.

4. Formulierung nach einem der Ansprüche 1 bis 3, enthaltend etwa 40 Gew.-%, basierend auf dem Gesamtgewicht der Formulierung, des Alkohols mit mehreren Hydroxylgruppen.

5. Formulierung nach einem der voranstehenden Ansprüche, worin der Alkohol mit mehreren Hydroxylgruppen ein Glykol oder Macrogol ist.

6. Formulierung nach Anspruch 5, worin das Glycol oder Macrogol Propylenglykol, Polyethylenglykol, Butan-1,3-diol oder Glycerin ist.

7. Formulierung nach einem der voranstehenden Ansprüche, worin die Ölphase mindestens einen Emulgator enthält.

8. Formulierung nach Anspruch 7, worin der Emulgator Ketostearylalkohol, Myristylalkohol, Glycerylmonostearat, Natriumlaurylsulfat, Polyoxyethylensorbitanmonostearat, Sorbitanmonostearat oder Sorbitanmonooleat ist.

9. Formulierung nach Anspruch 7, worin die Ölphase auch ein Fett und/oder ein Öl enthält.

10. Formulierung nach Anspruch 9, worin das Fett und/oder Öl Diisopropyladipat, Isocetylstearat, Propylenglykoldiester von Kokosnuß-Fettsäuren, Isopropylmyristat, Decyl-oleat, Isopropylpalmitat, Butylstearat, 2-Ethyl-hexylpalmitat, weißes Weichparaffin, flüssiges Paraffin, Mineralöle oder ein gemischter Ester von 2-Ethylhexansäure mit einem Gemisch von Cetyl- und Stearylalkoholen ist.

11. Äußerlich anzuwendende Öl-in-Wasser-Formulierung von 9-(2-Hydroxyethoxymethyl)-guanin oder eines pharmazeutisch verträglichen Salzes oder Esters davon, die eine dispergierte Ölphase und eine kontinuierliche wässrige Phase aufweist, dadurch gekennzeichnet, daß die wässrige Phase Wasser, mindestens 30 Gew.-%, basierend auf dem Gesamtgewicht der Formulierung, Propylenglykol und solubilisiertes Acyclovir oder dessen Salz oder dessen Ester enthält.

12. Verfahren zur Herstellung einer äußerlich anzuwendenden Formulierung nach einem der Ansprüche 1 bis 11, wobei die Kombination von Acyclovir, Alkohol mit mehreren Hydroxylgruppen und Wasser mit der Ölphase vermischt wird.

**Revendications**

1. Formulation topique huile-dans-eau de 9(2-hydroxyéthoxyméthyl)guanine (dite ci-après acyclovir) ou d'un sel ou ester pharmaceutiquement acceptable de celle-ci, comprenant une phase huileuse dispersée et une phase aqueuse continue, caractérisée en ce que la phase aqueuse comprend de l'eau, au moins 30% p/p, sur base du poids total de la formulation, d'un alcool polyhydroxylé miscible à l'eau et l'acyclovir ou son sel ou ester solubilisé.

2. Formulation suivant la revendication 1, qui comprend 1 à 10% p/p d'acyclovir ou un sel ou ester, 30% à 50% p/p de l'alcool polyhydroxylé, 20% à 40% p/p d'eau, tous les pourcentages étant sur base du poids total de la formulation, outre la phase huileuse.

3. Formulation suivant la revendication 1 ou 2, qui comprend 2 à 5% p/p d'acyclovir ou d'un sel ou ester, 35% à 45% p/p d'alcool polyhydroxylé, 25% à 40% p/p d'eau, tous les pourcentages étant sur base du poids total de la formulation, outre la phase huileuse.

4. Formulation suivant l'une quelconque des revendications 1 à 3, qui comprend environ 40% p/p, sur base du poids total de la formulation, de l'alcool polyhydroxylé.

5. Formulation suivant l'une quelconque des revendications précédentes, dans laquelle l'alcool polyhydroxylé est un glycol ou macrogol.

6. Formulation suivant la revendication 5, dans laquelle le glycol ou macrogol est le propylèneglycol, le polyéthylèneglycol, le butane-1,3-diol ou le glycérol.

7. Formulation suivant l'une quelconque des revendications précédentes, dans laquelle la phase huileuse comprend au moins un émulsionnant.

8. Formulation suivant la revendication 7, dans laquelle l'émulsionnant est l'alcool cétostéarylique, l'alcool myristylique, le monostéarate de glycéryle, le laurylsulfate de sodium, le monostéarate de polyoxyéthylène sorbitan, le monostéarate de sorbitan ou le mono-oléate de sorbitan.

9. Formulation suivant la revendication 7, dans laquelle la phase huileuse comprend aussi une graisse et/ou une huile.

10. Formulation suivant la revendication 9, dans lequelle la graisse et/ou huile sont l'adipate de diisopropyle, le stéarate d'isocétyle, le diester du propylèneglycol avec les acides gras de coprah, le myristate d'isopropyle, l'oléate de décyle, le palmitate d'isopropyle, le stéarate de butyle, le palmitate de 2-éthylhexyle, la paraffine blanche molle, la paraffine liquide, des huiles minérales ou un ester mixte d'acide 2-éthylhexanoïque avec un mélange d'alcools cétylique et stéarylique.

11. Formulation topique huile-dans-eau de 9-(2-hydroxyéthoxyméthyl)guanine ou d'un sel ou ester pharmaceutiquement acceptable de celle-ci comprenant une phase huileuse dispersée et une phase aqueuse continue, caractérisée en ce que la phase aqueuse comprend de l'eau, au moins 30% p/p, sur base du poids total de la formulation, de propylèneglycol et de l'acyclovir ou un sel ou ester de celui-ci solubilisé.

12. Procédé de préparation d'une formulation topique suivant l'une quelconque des revendications 1 à 11, qui comprend le mélange de la combinaison d'acyclovir, d'alcool polyhydroxylé et d'eau avec la phase huileuse.